(19) Europäisches Patentamt
European Patent Office
Office européen des brevets

(11) Veröffentlichungsnummer: **0 002 804**
**B1**

(12) # EUROPÄISCHE PATENTSCHRIFT

(21) Anmeldenummer: **78101798.3**

(22) Anmeldetag: **21.12.78**

(51) Int. Cl.³: **A 61 K 31/79,**
C 08 F 126/10,
C 08 G 18/63,
C 08 G 63/16, C 08 G 69/26

(54) **Biologisch abbaubare Vinylpyrrolidonpolymerisate, ihre Herstellung und Verwendung.**

(30) Priorität: **31.12.77 DE 2759150**

(43) Veröffentlichungstag der Anmeldung:
**11.07.79 Patentblatt 79/14**

(45) Bekanntmachung des Hinweises auf die Patenterteilung:
**25.03.81 Patentblatt 81/12**

(84) Benannte Vertragsstaaten:
**BE CH DE FR GB IT NL SE**

(56) Entgegenhaltungen:
**DE - C - 876 570**
**US - A - 3 216 983**

**CHEMICAL ABSTRACTS, Vol. 78, Nr. 30556p, 1973, Columbus, Ohio, USA, MIRSKOV R.G. et al.: "Poly(vinyl-pyrrolidone)derivatives," Seite 25**

(73) Patentinhaber: **BASF Aktiengesellschaft**
**Carl-Bosch-Strasse 38**
**D-6700 Ludwigshafen (DE)**

(72) Erfinder: **Straub, Ferdinand, Dr.**
**Ziegelstrasse 20**
**D-6832 Hockenheim (DE)**

(72) Erfinder: **Schenck, Hans-Uwe, Dr.**
**Erlenweg 6**
**D-6706 Wachenheim (DE)**

(72) Erfinder: **Lang, Siegfried, Dr.**
**Thomas-Mann-Strasse 22**
**D-6700 Ludwigshafen (DE)**

(72) Erfinder: **Brode, Egon, Dr.**
**Max-Liebermann-Strasse 10 b**
**D-6710 Frankenthal (DE)**

**EP 0 002 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Biologisch abbaubare Vinylpyrrolidonpolymerisate, ihre Herstellung und Verwendung

Für die erste Hilfe beim physiologischen Schock ist man häufig auf Blutersatzflüssigkeiten angewiesen, da Blutkonserven meist nicht sofort zur Verfügung stehen. Außerdem muß man ohne Verträglichkeitsrisiken (Blutgruppe, Rhesusfaktor) Blutflüssigkeit ersetzen können. Reine Salzlösungen (physiologische Kochsalzlösung, Ringerlösung) eignen sich wegen ihrer zu geringen Verweildauer im Gefäßsystem und wegen des fehlenden kolloidosmotischen Drucks (häufig wird anstelle dieses Ausdrucks onkotischer Druck gesagt) nicht zur Schockbehandlung.

Polyvinylpyrrolidon-Lösungen werden schon im großen Maßstab als Blutersatzflüssigkeit verwendet. Diese Polymeren sind wegen ihrer Ähnlichkeit zu Peptiden gut verträglich und ähneln bei entsprechend mittleren Molekulargewichten in ihrem kolloidosmotischen Druck dem des Blutplasma. Wenn ihr Molekulargewicht zu hoch ist, werden sie jedoch nicht vollständig ausgeschieden, sondern lagern sich im Reticuloendothel ab. Benutzt man andererseits als Blutersatzflüssigkeit ein Polymeres, das nur Moleküle mit niedrigem Molgewicht für eine gute Ausscheidbarkeit durch die Niere enthält, so ist der kolloidosmotische Druck und damit die Wirksamkeit gering. Polyvinylpyrrolidon ist ein sehr inertes Polymeres, da es radikalisch polymerisiert wird und die Polymerkette somit aus —C—C—Bindungen besteht. Die —C—C—Bindungen werden aber von den Enzymen des Körpers (Blut, Leber) nicht angegriffen und die Polymeren deshalb nicht abgebaut.

In der DE-PS 10 41 052 ist ein Verfahren zur Herstellung polypeptidartiger Verbindungen von physiologisch wirksamen Verbindungen beschrieben. Danach werden an ein Copolymeres aus Vinylpyrrolidon und Acrylsäure über Peptid-Brücken Wirkstoffe angekoppelt, die nach einiger Zeit in vivo von den Enzymen wieder abgespalten werden. Die Herstellung biologisch abbaubaren Polyvinylpyrrolidons durch Verknüpfung von Polyvinylpyrrolidon-Blöcken mit Peptideinheiten oder entsprechend mit Zuckereinheiten ist jedoch kostspielig und arbeitsintensiv.

Es wurde nun gefunden, daß Vinylpyrrolidonpolymere und -copolymere biologisch abgebaut werden, wenn mindestens ein

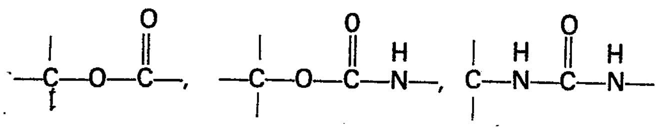 oder 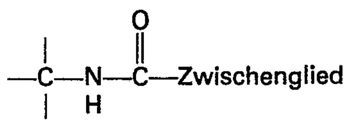

in der Polymerkette vorhanden ist. Es handelt sich um Vinylpyrrolidonpolymerisate, in denen 2 bis 50 Vinylpyrrolidonpolymerisat- oder Vinylpyrrolidoncopolymerisat-Blöcke vom Polymerisationsgrad 2 bis 1 000 durch Ester-, Amid-, Harnstoff- oder Urethangruppen enthaltende Zwischenglieder verknüpft sind und deren durchschnittliches Gesamtmolekulargewicht 1 000 bis 1 000 000 beträgt. Der Aufbau eines derartigen Vinylpyrrolidonpolymeren entspricht dem Blockpolymerschema

$$A — [B — A]_n — B — A$$

worin bedeuten

A Vinylpyrrolidonpolymer-oder copolymerblöcke mit einem Polymerisationsgrad zwischen 2 und 1 000

B kurzkettige Ester-, Amid-, Harnstoff- oder Urethangruppen enthaltende Zwischenglieder

n 0 bis 50.

Die Anzahl (n) der Blöcke aus Vinylpyrrolidonpolymerisat (A) und Ester-, Amid-, Harnstoff- oder Urethangruppen enthaltenden Zwischenglieder (B) hängt einerseits von dem Mol-Gewicht des Vinylpyrrolidonpolymerisats (A) ab und richtet sich andererseits nach dem gewünschten Mol-Gewicht des Blockpolymeren. Wenn das mittlere Mol-Gewicht des Vinylpyrrolidonpolymerisats niedrig ist (z.B. 500 bis 5 000) und man ein hohes Mol-Gewicht des Blockpolymeren wünscht (z.B. 50 000 bis 100 000), muß n groß sein. Bevorzugt für n ist 1 bis 20. Damit liegt der Gewichtsanteil von B am Blockpolymeren bei 1 bis 50% oder darüber.

Die Erfindung betrifft auch ein Verfahren, nach dem man aus niedrigmolekularen Vinylpyrrolidonpolymerisaten diese Polymere erhält, die gute kolloidosmotische Eigenschaften haben, im Körper nach einiger Zeit abgebaut und über die Niere voll ausgeschieden werden.

Zur Herstellung geht man von niedrigmolekularen Vinylpyrrolidonpolymeren mit funktionellen Endgruppen aus, die über Polykondensations- oder Polyadditionsreaktionen zu höhermolekularen Polymeren verknüft werden. Man verwendet ein bi-funktionelles Polymeres, das nach den üblichen Methoden hergestellt werden kann. Vinylpyrrolidonpolymere mit 2 funktionellen Endgruppen erhält man z.B. aus Vinylpyrrolidon durch Polymerisationsstart mit einem funktionelle Gruppen tragenden Polymerisationsinitiator unter Zusatz eines Reglers, der auch funktionelle Gruppen überträgt. Dies kann z.B. durch Polymerisation von Vinylpyrrolidon in wäßriger Lösung mit Wasserstoffperoxid oder durch Polymerisation nach DE-OS 25 14 127 erfolgen. Wenn man z.B. Amine wie Butylamin, Toluidin als Regler und Azobisisobutyroamidin als Initiator verwendet, erhält man ein Polyvinylpyrrolidon mit Amino-Endgruppen. Die erhaltenen Homopolymerisate des Vinylpyrrolidons werden schon lange als Hilfsmittel in der Medizin eingesetzt und brauchen deshalb nicht mehr toxikologisch geprüft werden. Es kann aber auch von Vorteil sein, Copolymere des Vinylpyrrolidons mit anderen Monomeren einzusetzen, wenn dabei das Prinzip der Bifunktionalität gewahrt wird. Monomere, die sich mit Vinylpyrrolidon copolymerisieren lassen sind z.B. Acrylsäure, Methacrylsäure, Acrylamid, Methacrylamid, Allylalkohol, Hydroxyäthylacrylat, Hydroxypropylacrylat, Butandiolmonoacrylat, Hydroxypropylacrylamid.

Eine weitere Möglichkeit, Polyvinylpyrrolidon mit OH-Endgruppen zu erhalten, besteht darin, daß man Polyvinylpyrrolidon, das durch radikalische Polymerisation von N-Vinylpyrrolidon-2 in Gegenwart von Wasserstoffperoxid als radikal bildendem Initiator erhalten worden ist, mit komplexen Hydriden behandelt, wobei — bezogen auf das Gewicht des Polymerisats — 0.1 bis 10, vorzugsweise 0.5 bis 5 % zur Anwendung gelangen. Als Hydride verwendet man vor allem solche, die wie Natrium- und Lithiumboranat wasserlöslich sind, doch läßt sich die Reaktion auch mit anderen wie $NaBH(OCH_3)_3$, $NaAlH_4$, $LiAlH_4$, $NaAlH_2(OCH_2OCH_3)_2$ oder LiAlH $[(OC(CH_3)_3]$ durchführen. Die stark reaktiven komplexen Hydride werden nur in solchen Mengen eingesetzt, daß die Lactamgruppe des Polyvinylpyrrolidons nicht angegriffen wird.

Die Reaktion mit den komplexen Hydriden wird, wenn möglich, in Wasser durchgeführt, was mit Lithium- und Natriumboranat realisierbar ist. Bei den anderen verwendet man zweckmäßigerweise Lösungsmittel wie niedere Alkohole, z.B. Methanol, Äthanol, Iso- und n-Propanol, n-Butanol- oder tert. Butanol, Äther, wie Dioxan oder Tetrahydrofuran, oder Aromaten wie Benzol, Toluol oder Xylol. Die Reaktion wird bei Temperaturen von 1 bis 150, vorzugsweise 15 bis 80°C durchgeführt. Sie richtet sich nach dem Siedepunkt der Lösungsmittel. Wenn man in wäßrigen oder alkoholischen Lösungsmitteln arbeitet, stellt man im allgemeinen pH-Werte von ca. 7 vor der Reaktion ein. Die Dauer der Reaktion schwankt zwischen 0,5 und 24, vorzugsweise 2 bis 8 Stunden. Falls das Polyvinylpyrrolidon Carboxylgruppen enthält, kann es vorteilhaft sein, die Carboxylgruppen vor der Umsetzung mit Hydriden nach den üblichen Methoden zu verestern.

Um eventuell im Polymerisat vorhandene Verunreinigungen zu entfernen, hat es sich als sehr nützlich erwiesen, die Polymerisatlösung über Ionenaustauscher zu reinigen. Geeignet sind z.B. Ionenaustauscher auf Polystyrolbasis mit Sulfo, Carboxylgruppen oder mit quaternären Ammoniumgruppen oder auf Basis von sauren oder basischen Silikaten. Hiervon sind die unter den Warenzeichen AMBERLITE (Rohm & Haas Comp.) und LEWATIT (Bayer AG) vertriebenen und im Handel erhältlichen Produkte zu nennen.

Besonders bevorzugt ist eine Reinigung im Anschluß an die Umsetzung mit den komplexen Hydriden, da dadurch auch durch diese Umsetzung entstehende Verunreinigungen mit entfernt werden. Auf diese Weise zusätzlich behandelte Polymerisate zeigen bei der Polykondensation bzw. Polyaddition eine wesentlich erhöhte Reaktivität gegenüber den ungereinigten Polymerisaten.

Als funktionelle Gruppen wählt man zweckmäßigerweise Hydroxyl- oder Aminogruppen. Die funktionellen Gruppen kann man auch durch Copolymerisierung des Vinylpyrrolidons mit funktionelle Gruppen tragenden Comonomeren einführen. Beispiele dieser Comonomeren sind im vorangehenden Absatz genannt. Die Funktionellen Gruppen wird man vorteilhaft danach auswählen, was für ein Zwischenglied man einbauen möchte. Für Ester- oder Urethan-Zwischenglieder wird man bevorzugt ein Polymeres mit Hydroxyl-Endgruppen, für Amid- oder Harnstoff Zwischenglieder. Amino-Endgruppen verwenden.

Die Mol-Gewichte bzw. den Polymerisationsgrad des Polymeren wählt man so, daß es nach Abbau des erfindungsgemäßen Blockpolymeren im Körper gut über die Nieren ausgeschieden werden kann. Der Polymerisationsgrad liegt daher zwischen 2 und 1 000, bevorzugt zwischen 10 bis 300.

Für die Bildung der Einheiten B, den Ester-, Amid-, Harnstoff- oder Urethangruppen enthaltenden Zwischengliedern in den erfindungsgemäßen Blockpolymeren, verwendet man bevorzugt bifunktionelle Carbonsäurederivate oder Isocyanate, um eine gute Verknüpfung zu gewährleisten. Je nach Wahl der Carbonsäurederivate bzw. der Isocyanate kann man die Anzahl der spaltbaren Bindungen und die Bindungsart festlegen. Damit ergibt sich die Möglichkeit, die Geschwindigkeit des enzymatischen Abbaues einzustellen. Für die Zwischenglieder kommen beispielsweise die Säurehalogenide von Dicarbonsäuren in Betracht, wie Phosgen, Oxalylchlorid, Malondichlorid, Bernsteindichlorid, Glutardichlorid, Adipindichlorid, Sebacindichlorid, Phthalsäuredichlorid und Terephthalsäuredichlorid oder Diisocyanate, wie Toluylendiisocyanat, Hexamethylendiisocyanat, Methylenbiscyclohexylendiisocyanat und Bis(3-methyl-4-isocyanatocyclohexyl)-methan.

Wie die Erläuterung zu den Polymerisatblöcken A und den sie verknüpfenden Zwischengliedern B zeigen, erhält man

Esterzwischenglieder von solchen Polymerisaten, die Hydroxylgruppen tragen, mit Dicarbonsäurederivaten,

Amidzwischenglieder von solchen Polymerisaten, die Aminogruppen tragen, mit Dicarbonsäurederivaten,

Urethanzwischenglieder von solchen Polymerisaten, die Hydroxylgruppen tragen, mit Diisocyanaten,

Harnstoffzwischenglieder von solchen Polymerisaten, die Aminogruppen tragen, mit Diisocyanaten.

Es werden demnach in den Zwischengliedern B jeweils zwei Ester-, Amid-, Urethan- oder Harnstoffgruppen durch z.B. eine einfache Bindung (Oxalylchlorid) oder eine Methylengruppe (Malondichlorid) verknüpft. Bei Verwendung von Phosgen ist für

— A — B — A — B zu setzen — A — OCOO — A — OCOO — ,

d.h. die Estercarbonylgruppe ist in jedem B nur einmal vorhanden.

Im allgemeinen können die Zwischenglieder B z.B. von aliphatischen Dicarbonsäuren oder Diisocyanaten stammen, in denen die Carboxylgruppen durch eine Kette von 0 bis 10 Kohlenstoffatomen oder die beiden Isocyanatgruppen durch eine Kette von 1 bis 10 Kohlenstoffatomen verbunden sind.

Die Verknüpfung der Blöcke A über die Einheiten B, d.h. die Polykondensation bzw. Polyaddition zwischen dem bi- oder mehrfunktionellen Vinylpyrrolidonpolymerisat und den bifunktionellen Carbonsäurederivaten bzw. Isocyanaten führt man vorzugsweise in Lösung aus. Sie soll vorzugsweise wasserfrei sein, damit die zur Verknüpfung benutzten Säurederivate bzw. Isocyanate keine unerwünschten Nebenprodukte bilden. Als Lösungsmittel können solche verwendet werden, die Vinylpyrrolidonpolymerisate gut lösen und selbst keine Reaktionen mit den Carbonsäurederivaten oder den Isocyanaten eingehen. Bevorzugt sind aprotische Lösungsmittel, wie N-Methylpyrrolidon, Dimethylsulfoxid, Dimethylformamid, Toluol und chlorierte Kohlenwasserstoffe.

Für die Umsetzung kann man die Komponente A und die für die Bildung des Zwischengliedes B erforderliche Komponente zusammengeben oder eine Komponente zur Lösung der anderen zudosieren.

Nach der Umsetzung kann man das Lösungsmittel durch Abdestillieren, Wasserdampfdestillieren oder durch Ausfällen des Blockpolymeren entfernen.

Die Blockpolymeren dieser Erfindung zeigen gute kolloidosmotische Effekte, werden biologisch abgebaut, und die dadurch entstehenden niedrigmolekularen Verbindungen werden leicht über die Nieren ausgeschieden. Ihre Verträglichkeit ist so gut wie die der entsprechenden Vinylpyrrolidonpolymerisate ohne Zwischenglieder. Sie eignen sich daher gut als pharmazeutische Hilfsmittel und als Plasmaexpander. Die Abbauzeit unter der Einwirkung von Körper-enzymen beträgt Stunden bis Tage. Diese Eigenschaften sind sehr überraschend, weil synthetische Polyester, Polyurethane und Polyamide als Implantate jahrelang ohne größeren Abbau im Körper verbleiben.

Die fologenden Beispiele veranschaulichen die vorliegende Erfindung, ohne sie zu beschränken. Die angegebenen K-Werte werden gemessen nach H. Fikentscher, Cellulosechemie 13, 58—64, 71—74 (1932). Aus dem K-Wert wurde über die Gleichung $M_w = 1{,}9 \times (\text{K-Wert})^3$ das Molekulargewicht berechnet.

Beispiele

1a) 100 g Polyvinylpyrrolidon mit K-Wert 14, hergestellt durch Polymerisation von Vinylpyrrolidon in waßriger Lösung mit Wasserstoffperoxid als Starter und Entfernen des Pyrrolidons, werden in 300 ml Chloroform gelöst; Wasser wird durch Kreislaufdestillation entfernt. Man läßt die Lösung abkühlen und versetzt sie langsam bei Raumtemperatur mit 12 ml Oxalylchlorid. Die Lösung wird deutlich viskos, die Farbe leicht braun. Man rührt noch 4 Stunden bei Raumtemperatur, neutralisiert mit Natriumhydrogencarbonat und fällt das Polymere durch Eintropfen des Reaktionsgemisches in 2 l Äther aus. Das Polymere hat einen K-Wert von 17,6 (5%ig in Wasser), entsprechend einem berechneten Molekulargewicht von 10 358.
Zur Entfernung des Pyrrolidons aus dem Ausgangspolymerisat kann man beispielsweise über 2 kg Lewatit S 100 reinigen, worauf man gefriertrocknet und dann in den 300 ml Chloroform löst.

1b) 3 g des nach 1a) erhaltenen Polymeren werden in 100 ml entsalztem Wasser und 100 ml Blutplasma bei 37° und pH 7,2 16 Stunden inkubiert. Danach wird gefriergetrocknet, das Pulver wird in Methylenchlorid/Methanol aufgeschlämmt, abfiltriert und die Lösung in 200 ml Äther ausgefällt. Man erhält ein Polymeres mit K-Wert 14,2.

2a) 100 g Polyvinylpyrrolidon mit K-Wert 12, hergestellt durch Polymerisation nach DE-OS 25 14 127, werden in 300 ml Toluol gelöst; Wasser wird durch Kreislaufdestillation entfernt. Die abgekühlte Lösung wird mit 9 ml Hexamethylendiisocyanat versetzt. Man läßt über Nacht stehen, versetzt mit wenig Äthanol und destilliert mittels Wasserdampf die organischen Lösungsmittel ab. Die entstehende wäßrige Lösung ist leicht trübe. Der K-Wert des Polymeren beträgt 17,7.

2b) Wie in 1b) beschrieben, wird die nach 2a) erhaltene Lösung inkubiert. Man erhält ein Polymeres mit K-Wert 12.

3a) 100 g eines Copolymeren von Vinylpyrrolidon mit 2-Hydroxypropylacrylat (95:5) mit K-Wert 18,1 werden in 300 ml Dichloräthan gelöst. Zu der Lösung tropft man langsam 9 ml Adipinsäuredichlorid bei Raumtemperatur und läßt über Nacht stehen. Man neutralisiert zweckmäßigerweise mit Natriumhydrogencarbonat. Danach fällt man das Polymere durch Eintropfen des Reaktionsgemisches in 2 l Äther aus. Das Pulver hat einen K-Wert von 24,3, entsprechend einem berechneten Molekulargewicht von 27 262.

3b) Wie in 1b) beschrieben, wird das nach 3a) erhaltene Polymere inkubiert. Nach der Inkubation hat das Polymere einen K-Wert von 19,3.

4a) 100 g Polyvinylpyrrolidon mit K-Wert 14,3 hergestellt nach DE-OS 25 14 127 in Äthanol als Lösungsmittel, werden in 300 ml Methyenchlorid gelöst; Wasser wird durch Kreislaufdestillation entfernt. Die abgekühlte Lösung wird mit 12 ml Bernsteinsäuredichlorid versetzt. Man läßt über Nacht stehen und fällt das Polymere durch Eingießen in 2 l Äther aus. Man löst das Polymere in 200 ml Wasser und neutralisiert mit Natriumhydrogencarbonat, es zeigt einen K-Wert von 16,2, was einem berechneten Molekulargewicht von 8 078 entspricht.

4b) Wie in 1b) beschrieben, wird die nach 4a) erhaltene Lösung inkubiert. Man erhält ein Polymeres mit K-Wert 14,8.

5a) 100 g eines Copolymeren von Vinylpyrrolidon mit 2-Hydroxypropylacrylat (95:5) mit einem K-Wert von 14,4 werden in 300 g Chloroform gelöst, Wasser wird durch Kreislaufdestillation entfernt. Nach dem Abkühlen wird mit 12 g Sebacindichlorid versetzt und über Nacht stehen gelassen. Es wird mit Natriumhydrogencarbonat neutralisiert und das Polymere durch Eintropfen in 2 l Äther ausgefällt. Das Polymerisat hat einen K-Wert von 17,1 (5%ig in Wasser), was einem berechneten Molgewicht von 9 500 entspricht.

5b) Wie in 1b) beschrieben, wird das Produkt 5a) inkubiert. Nach der Inkubation hat das Polymerisat einen K-Wert von 16,7.

6a) 100 g eines Copolymeren von Vinylpyrrolidon mit 2-Hydroxypropylacrylat (92,5 : 7,5) mit einem K-Wert von 13,5 werden in 300 g Dimethylformamid gelöst. 100 g Dimethylformamid werden in Vakuum abdestilliert. Nach dem Abkühlen versetzt man mit 12 g Toluylendiisocyanat und rührt 4 Stunden lang. Das Polymere wird durch Eingießen in 2 l Äther ausgefällt und hat einen K-Wert von 21,3 (5%ig in Wasser), was einem berechneten Molgewicht von 18 360 entspricht.

6b) Wie in 1b) beschrieben, wird das Produkt 6a) sechs Tage lang inkubiert. Nach der Inkubation hat das Produckt einen K-Wert von 16,1.

7a) 100 g Polyvinylpyrrolidon mit K-Wert 17, hergestellt durch Polymerisation von Vinylpyrrolidon in wäßriger Lösung mit Wasserstoffperoxid als Initiator, werden in wäßriger Lösung mit 2 g $NaBH_4$ versetzt und während des Schäumens gerührt. Danach reinigt man die Lösung über 2 kg Lewatit S 100 und Lewatit M 500. Das gefriergetrocknete Polymerisat wird in 300 g Dimethylformamid gelöst. 100 g Dimethylformamid werden im Vakuum abdestilliert. Man läßt die Lösung abkühlen und versetzt mit 12 g Hexamethylendiisocyanat. Man läßt über Nacht stehen und fällt das Polymerisat durch Eingeißen in 2 l Äther aus. Es hat einen K-Wert von 27,9 (5%ig in Wasser), was einem berechneten Molgewicht von 41 263 entspricht.

7b) Wie in 1b) beschrieben, wird das Produkt von 7a) sechs Tage inkubiert. Nach der Inkubiert hat es einen K-Wert von 24,6.

8a) 100 g Polyvinylpyrrolidon mit K-Wert 17, hergestellt durch Polymerisation von Vinylpyrrolidon mit Wasserstoffperoxid als Initiator, werden in wäßriger Lösung mit 2 g $NaBH_4$ versetzt und während des Schäumens gerührt. Danach reinigt man die Lösung über 2 kg Lewatit S 100 und Lewatit M 500. Das gefriergetrocknete Polymerisat wird in 300 g Chloroform gelöst. Wasser wird durch Kreislaufdestillation entfernt, man läßt abkühlen und versetzt mit 12 g Adipinsäuredichlorid. Man rührt 4 Stunden, fällt das Polymere durch Eingießen in 2 l Äther aus und löst es in 200 g Wasser. Nach der Neutralisation mit $NaHCO_3$ hat das Polymerisat einen K-Wert von 23,4, was einem berechneten Molgewicht von 24 344 entspricht.

8b) Wie in 1b) beschrieben, wird das Produkt von 8a) inkubiert. Nach der Inkubation hat es einen K-Wert von 19,1.

### Vergliechsbeispiel 1

Polyvinylpyrrolidon mit K-Wert 14, polymerisiert in Wasser mit Wasserstoffperoxid wurde wie in 1b) beschrieben inkubiert. Nach der Inkubation war der K-Wert immer noch 14.

### Vergleichsbeispiel 2

Polyvinylpyrrolidon mit K-Wert 12, hergestellt durch Polymerisation nach DE-OS 25 14 127, wurde wie in 1b) beschrieben inkubiert. Nach der inkubation hatte das Produkt K-Wert 12,4.

### Vergleichsbeispiel 3

Ein Copolymeres von Vinylpyrrolidon mit 2-Hydroxypropylacrylat (95:5) mit K-Wert 18,1 wurde wie in 1b) beschrieben inkubiert. Danach hatte das Polymere immer noch K-Wert 18,1.

### Vergleichsbeispiel 4

Polyvinylpyrrolidon mit K-Wert 14,3, hergestellt durch Polymerisation nach DE-OS 25 14 127, wurde wie in 1b) beschrieben inkubiert. Nach Inkubation hatte das Produkt K-Wert 14,4.

### Vergleichsbeispiel 5

Polyvinylpyrrolidon mit dem K-Wert 17, polymerisiert in Wasser mit Wasserstoffperoxid, um-

gesetzt mit $NaBH_4$ und gereinigt über Ionenaustauscher, wird wie in 1b) beschrieben inkubiert. Danach hatte das Polymere K-Wert 17,0.

Zur Herstellung von Blutersatzflüssigkeiten können die üblichen Methoden zur Bereitung pyrogenfreier Flüssigkeiten angewendet, auch physiologische Kochsalzlösung, Ringerlösung zugemischt werden.

Als Richtlinie für Blutersatzflüssigkeiten können z.B. folgende Angaben dienen:

| | |
|---|---|
| a) biologisch abbaubares Polymerisat | 4,0 g |
| Elektrolytlösung | ad 100 ml |

Zusammensetzung der Elektrolytlösung:

0,7 g NaCl, 0,042 g KCl, 0,05 g $CaCl_2$, 0,0005 g $MgCl_2$, 0,025 g $NaHCO_3$, Wasser pro inj. ad 100 ml.

Zur Herstellung werden 4 g des Polymeren unter Rühren in der Elektrolytlösung aufgelöst. Die Lösung gibt man über ein Filter, um Schwebeteilchen zu entfernen, und sterilisiert bei 120°C 20 Minuten;

oder

| | |
|---|---|
| b) biologisch abbaubares Polymerisat | 4,0 g |
| physiologische Kochsalzlösung | 100 ml |

Filtration der hergestellten Lösung und anschließend Sterilisation bei 120°C 20 Minuten.

Der Zusatz von Elektrolyt- oder Salzlösungen empfiehlt sich, wenn man den osmotischen Druck der Blutersatzflüssigkeit regulieren oder modifizieren will.

**Patentansprüche**

1. Biologisch abbaubare Vinylpyrrolidonpolymerisate des Aufbauschemas

$$A — [B — A]_n — B — A$$

worin bedeuten
- A Vinylpyrrolidonpolymer- oder copolymerblöcke mit einem Polymerisationsgrad zwischen 2 und 1 000,
- B kurzkettige Ester-, Amid-, Harnstoff- oder Urethangruppen enthaltende Zwischenglieder
- n 0 bis 50.

und deren durchschnittliches Gesamtmolekulargewicht 1 000 bis 1 000 000 beträgt.

2. Vinylpyrrolidonpolymerisate nach Anspruch 1, in denen Hydroxylgruppen tragende Vinylpyrrolidonpolymerisat-Blöcke durch Zwischenglieder von aliphatischen Dicarbonsäuren mit einer Kette von 0 bis 10 Kohlenstoffatomen zwischen den beiden Carbonylgruppen verbunden sind.

3. Vinylpyrrolidonpolymerisate nach Anspruch 1, in denen Hydroxylgruppen tragende Vinylpyrrolidonpolymerisat-Blöcke durch Zwischenglieder von aliphatischen Diisocyanaten mit einer Kette von 2 bis 10 Kohlenstoffatomen Zwischen den beiden Isocyanatgruppen verbunden sind.

4. Vinylpyrrolidonpolymerisate nach Anspruch 2, deren Hydroxylgruppen tragender Vinylpyrrolidonpolymerisat-Block ein Copolymerisat von Vinylpyrrolidon mit geringen Mengen eines Hydroxylgruppen tragenden Comonomeren ist.

5. Verfahren zur Herstellung von Vinylpyrrolidonpolymerisaten gemäß Anspruch 1, dadurch gekennzeichnet, daß man Hydroxyl- oder Amino-Endgruppen tragende Vinylpyrrolidonpolymerisate mit einem Polymerisationsgrad von 2 bis 1 000 in Abwesenheit von Wasser mit mehrfunktionellen Carbonsäurederivaten in einem aprotischen Lösungsmittel umsetzt.

6. Verfahren nach Anspruch 5, dadurch gekennzeichnet, daß man als Carbonsäurederivate bifunktionelle Carbonsäurehalogenide benutzt.

7. Verfahren zur Herstellung von Vinylpyrrolidonpolymerisaten gemäß Anspruch 1, dadurch gekennzeichnet, daß man Hydroxyl- oder Amino-Endgruppen tragende Vinylpyrrolidonpolymerisate mit einem Polymerisationsgrad von 2 bis 1 000 in Abwesenheit von Wasser mit mehrfunktionellen Isocyanaten in einem aprotischen Lösungsmittel umsetzt.

8. Verwendung von Vinylpyrrolidonpolymerisaten gemäß einem der Ansprüch 1 bis 4 für die Herstellung von Blutersatzflüssigkeiten.

9. Bluttersatzflüssigkeit, enthaltend ein Vinylpyrrolidonpolymerisat gemäß einem der Ansprüche 1 bis 4.

**Revendications**

1. Polymère de vinylpyrrolidone dégradable biologiquement, de schéma de structure:

$$A — [B — A]_n — B — A$$

dans laquelle
A représente des blocs de polymère ou copolymère de vinylpyrrolidone d'un degré de polymérisation compris entre 2 et 1 000,
B une terme intermédiaire contenant des groupes ester, amide, urée ou uréthane à courte chaîne
n est un nombre de 0 à 50,
et dont le poids moléculaire moyen est compris entre 1 000 et 1 000 000.

2. Polymère de vinylpyrrolidone selon la revendication 1, dans lequel des blocs de polymère de vinylpyrrolidone portant des groupes hydroxyle sont reliés, par des termes intermédiaires d'acides dicarboxyliques aliphatiques à chaîne de 0 à 10 atomes de carbone, entre les deux groupes carbonyle.

3. Polymère de vinylpyrrolidone selon la revendication 1, dans lequel des blocs de polymère de vinylpyrrolidone portant des groupes hydroxyle sont reliés, par des termes intermédiaires de diisocyanates aliphatiques à chaîne de 2 à 10 atomes de carbone, entre les deux groupes isocyanate.

4. Polymère de vinylpyrrolidone selon la revendication 2, dont le bloc de polymère de vinylpyrrolidone portant des groupes hydroxyle est un copolymère de vinylpyrrolidone avec de faibles quantités d'un comonomère portant des groupes hydroxyle.

5. Procédé de préparation de polymères de vinylpyrrolidone selon la revendication 1, caractérisé par le fait que l'on fait réagir dans un solvant aprotique des polymères de vinylpyrrolidone portant des groupes terminaux hydroxyle ou amino d'un degré de polymérisation de 2 à 1 000, en l'absence d'eau, avec des dérivés d'acides carboxyliques polyfunctionnels.

6. Procédé selon la revendication 5, dans lequel on utilise comme dérivés d'acide carboxylique des halogénures d'acide carboxylique bifonctionnels.

7. Procédé pour la préparation de polymères de vinylpyrrolidone selon la revendication 1, dans lequel on fait réagir dans un solvant aprotique des polymères de vinylpyrrolidone portant des groupes hydroxyle ou amino d'un degré de polymérisation de 2 à 1 000, en l'absence d'eau, avec des isocyanates polyfonctionnels.

8. Utilisation de polymères de vinylpyrrolidone selon l'une des revendications 1 à 4 pour la préparation de liquides de substitution du sang.

9. Liquides de substitution du sang contenant un polymère de vinylpyrrolidone selon l'une des revendications 1 à 4.

**Claims**

1. A biodegradable vinylpyrrolidone polymer of the formula

$$A — [B — A]_n — B — A$$

where A is a vinylpyrrolidone polymer or copolymer block having a degree of polymerization of from 2 to 1,000, B is a short-chain connecting unit containing ester, amide, urea or urethane groups and n is from 0 to 50, and which has an average total molecular weight of from 1,000 to 1,000,000.

2. A vinylpyrrolidone polymer as claimed in claim 1, wherein vinylpyrrolidone polymer blocks carrying hydroxyl groups are linked by connecting units of aliphatic dicarboxylic acids having a chain of from 0 to 10 carbon atoms between the two carbonyl groups.

3. A vinylpyrrolidone polymer as claimed in claim 1, wherein vinylpyrrolidone polymer blocks carrying hydroxyl groups are linked by connecting units of aliphatic diisocyanates having a chain of from 2 to 10 carbon atoms between the two isocyanate groups.

4. A vinylpyrrolidone polymer as claimed in claim 2, wherein the vinylpyrrolidone polymer block carrying hydroxyl groups is a copolymer of vinylpyrrolidone with small amounts of a comonomer carrying hydroxyl groups.

5. A process for the manufacture of a vinylpyrrolidone polymer as claimed in claim 1, characterized in that vinylpyrrolidone polymers carrying hydroxyl or amino end groups and having a degree of polymerization of from 2 to 1,000 are reacted with polyfunctional carboxylic acid derivatives in an aprotic solvent in the absence of water.

6. A process as claimed in claim 5, characterized in that bifunctional carboxylic acid halides are used as carboxylic acid derivatives.

7. A process for the manufacture of a vinylpyrrolidone polymer as claimed in claim 1, characterized in that vinylpyrrolidone polymers carrying hydroxyl or amino end groups and having a degree of polymerization of from 2 to 1,000 are reacted with polyfunctional isocyanates in an aprotic solvent in the absence of water.

8. Use of a vinylpyrrolidone polymer as claimed in any of claims 1 to 4 for the preparation of a plasma substitute.

9. A plasma substitute containing a vinylpyrrolidone polymer as claimed in any of claims 1 to 4.